# DEMANDE DE BREVET EUROPEEN

(11) **EP 4 796 141 A1**
(43) Date de publication de la demande: **26.08.2026**
(21) Numéro de dépôt: 26158813.1
(22) Date de dépôt: 16.02.2026
(51) Int. Cl.: A61M 3/02, A61J 1/06, A61J 1/14

(54) **KIT POUR IRRIGATION NASALE ET PROCÉDÉ DE PRÉPARATION D'UN DISPOSITIF POUR IRRIGATION NASALE À PARTIR D'UN TEL KIT**

(30) Priorité: 21.02.2025 FR 2501825
(71) Demandeur: Adalyo, 75008 PARIS (FR)
(72) Inventeur: BATTEUR, Matthieu, 49100 ANGERS (FR); BONNET, Johan, 44210 PORNIC (FR)
(74) Mandataire: AtlantIP International

(57) **Abrégé**

L'invention concerne un kit (1) pour irrigation nasale, ledit kit (1) comprenant :
- un dispositif d'injection (3) comprenant un embout de décharge (3a) ;
- un embout nasal (5) comprenant un canal (7) débouchant à deux extrémités (5a, 5b) opposées dudit embout (5), ledit embout (5) étant configuré pour que l'embout de décharge (3a) dudit dispositif d'injection (3) puisse être inséré dans ledit canal (7) ;
- un récipient (9) de liquide de nettoyage nasal qui comprend un orifice de distribution ;
caractérisé en ce que ledit un orifice est configuré, d'une part, pour présenter une coopération de forme avec l'embout de décharge (3a), et d'autre part, pour présenter un diamètre inférieur à chacune des extrémités (5a, 5b) dudit embout nasal (5) où débouche ledit canal (7).

## Description

La présente invention se rapporte au domaine général de l'irrigation nasale, et plus particulièrement aux dispositifs et procédés permettant d'injecter un liquide (par exemple nettoyant) dans la cavité nasale d'un individu. La présente invention se rapporte plus particulièrement à un kit comprenant tous les éléments nécessaires à la pratique d'une irrigation nasale.

L'irrigation nasale ou lavage nasal consiste ainsi à l'injection ou introduction dans l'une des narines du nez d'un liquide nettoyant, tel qu'une solution isotonique ou physiologique, puis en l'évacuation dudit liquide par l'autre narine ou la cavité buccale.

Un tel procédé permet de nettoyer la cavité nasale et ainsi de dégager les voies aériennes, tout en étant également un traitement d'appoint de la sinusite aigüe ou chronique, des infections virales des voies aériennes supérieures ou encore de la rhinite allergique.

L'irrigation nasale est avantageusement utilisée dans le cadre du nettoyage des cavités nasales d'enfants, notamment lorsque les enfants sont malades et des mucosités encombrent leurs voies respiratoires et nécessitent d'être désobstruées.

L'injection d'un tel liquide dans la cavité nasale est généralement réalisée au moyen d'un dispositif d'injection adéquat, tel qu'une seringue (sans aiguille), une poire à lavement, un pot « neti », etc., ainsi que d'une bouteille contenant le liquide de nettoyage nasal.

L'une des problématiques est que la plupart des gens utilisent le dispositif d'injection pour réaliser le prélèvement du liquide et réaliser l'injection dans une cavité nasale, sans que celui-ci soit nettoyé après usage, ledit dispositif d'injection est donc contaminé et lors d'une réutilisation celui-ci va souiller le liquide de nettoyage nasal et favoriser des infections par l'utilisation d'un dispositif d'injection et/ou de liquide qui ne sont plus sains, voire stériles.

Il est donc nécessaire de trouver une solution qui favorise l'application de règles d'hygiène usuelles et empêche l'utilisation de matériel contaminé et/ou sale pour réaliser une irrigation nasale, voire empêche la contamination du dispositif d'injection et/ou du liquide de nettoyage nasal. Il est également nécessaire de trouver une solution permettant de réduire la perte de liquide de nettoyage nasal lors de la préparation des éléments nécessaires à la réalisation d'une irrigation nasale.

La présente invention se propose ainsi de remédier à au moins un des inconvénients précités en proposant un nouveau type de kit pour irrigation nasale, ledit kit comprenant :
- un dispositif d'injection comprenant un embout de décharge ;
- un embout nasal comprenant un canal débouchant à deux extrémités opposées dudit embout nasal, ledit embout nasal étant configuré pour que l'embout de décharge dudit dispositif d'injection puisse être inséré dans ledit canal ;
- un récipient de liquide de nettoyage nasal qui comprend un orifice de distribution ; caractérisé en ce que ledit orifice est configuré, d'une part, pour présenter une coopération de forme avec l'embout de décharge, et d'autre part, pour présenter un diamètre inférieur à chacune des extrémités dudit embout nasal où débouche ledit canal.

La présente invention se présente donc sous forme d'un kit comprenant différents éléments nécessaires à la préparation d'un dispositif d'injection pour procéder à une irrigation nasale. Plus particulièrement, l'orifice de distribution du récipient est configuré pour ne pouvoir coopérer qu'avec l'embout de décharge du dispositif d'injection, puis par la suite pour que l'embout nasal soit fixé sur l'embout de décharge du dispositif d'injection avant utilisation pour une irrigation nasale, évitant ainsi de salir l'embout de décharge lors la préparation et de l'irrigation nasale en elle-même.

L'ensemble des éléments dudit kit selon l'invention permettent donc de préparer un dispositif d'injection et d'injecter le liquide dans une cavité nasale sans contaminer le dispositif d'injection et/ou le liquide de nettoyage nasal. Par ailleurs, le kit selon l'invention permet de limiter la perte de liquide de nettoyant nasal lors de la préparation d'un dispositif d'injection pour irrigation nasale.

De plus, ledit kit selon l'invention présente l'avantage d'être peu onéreux et de nécessiter peu de modifications sur des éléments déjà existants, tout en présentant une configuration qui limite les possibilités qu'un utilisateur réalise une préparation pour irrigation nasale, ainsi qu'une irrigation nasale, dans de mauvaises conditions d'hygiène.

Selon une caractéristique possible, ledit récipient comprend un col ouvert obturé par un opercule, ledit orifice de distribution étant aménagé dans ledit opercule.

De manière avantageuse, l'orifice d'accès au liquide de nettoyage nasal est aménagé sur un opercule qui vient obturer totalement l'ouverture du col du récipient, limitant ainsi les risques de contaminations extérieures. De plus, les dimensions de l'orifice peuvent être adaptées en fonction des dimensions de l'embout de décharge dudit dispositif d'injection, évitant ainsi que n'importe quel dispositif d'injection soit utilisé avec ledit récipient, notamment des dispositifs inadaptés.

Selon une autre caractéristique possible, ledit opercule comprend une paroi, ledit orifice de distribution étant aménagé dans ladite paroi, la paroi présentant une épaisseur inférieure ou égale à la longueur dudit embout de décharge dudit dispositif d'injection.

La longueur de l'embout de décharge est avantageusement supérieure à l'épaisseur de la paroi de l'opercule, permettant ainsi qu'une partie de l'embout de décharge soit située dans le récipient de liquide de nettoyage nasal pour faciliter le remplissage dudit dispositif de liquide.

Selon une autre caractéristique possible, ledit opercule est amovible.

Ledit opercule est configuré pour pouvoir être fixé de façon amovible sur le récipient, et plus particulièrement dans le col ouvert dudit récipient, ceci afin par exemple de pouvoir être retiré pour transvaser plus facilement le liquide contenu dans ledit récipient dans un autre contenant.

Selon une autre caractéristique possible, ledit opercule est monté de façon étanche sur ledit récipient.

De manière avantageuse, ledit opercule obture l'ouverture du col du récipient de manière étanche, notamment pour permettre de retourner le récipient lors du remplissage du dispositif d'injection, facilitant ainsi le transfert du liquide du récipient au dispositif d'injection.

Selon une autre caractéristique possible, ledit orifice présente une longueur d'au moins 0,5 mm, et présente par exemple une longueur comprise entre 0,5 et 10 mm.

On notera qu'on entend par longueur de l'orifice, ou encore profondeur de l'orifice, la direction sensiblement orthogonale à la section de passage dudit orifice.

Selon une autre caractéristique possible, ledit embout de décharge dudit dispositif d'injection est configuré pour être emmanché en force dans le canal de l'embout nasal et dans l'orifice de distribution.

De manière avantageuse, l'emmanchement en force de l'embout permet de décrocher les éléments contaminants (tels que des saletés, de la poussière, etc.) qui peuvent être situés sur ledit embout de décharge, limitant (voire empêchant) ainsi l'insertion d'éléments contaminants dans le liquide dudit récipient.

Selon une autre caractéristique possible, ledit embout de décharge dudit dispositif d'injection et l'orifice de distribution dudit récipient comprennent respectivement un taraudage et un filetage complémentaires, lesdits embout de décharge et orifice étant configurés pour être vissés l'un à l'autre.

Selon une autre caractéristique possible, ledit orifice de distribution présente une section progressive, la partie la plus évasée étant orientée vers l'extérieur dudit récipient.

Le fait que l'orifice présente une section évasée, notamment vers l'extérieur du récipient, permet de faciliter le guidage de l'embout de décharge dans l'orifice dudit récipient, simplifiant entre autres l'emmanchement en force dudit embout du dispositif d'injection dans ledit orifice.

Selon une autre caractéristique possible, ledit récipient comprend un bouchon qui est configuré pour être fixé sur le col dudit récipient, ledit bouchon comprenant un pion d'obturation qui est configuré pour obturer l'orifice de distribution dudit opercule lorsque le bouchon est fixé audit col.

Le récipient comprend avantageusement un bouchon qui peut fermer ou non le col, et plus particulièrement l'orifice de distribution dudit récipient, l'obturation de l'orifice par un pion permettant de limiter la contamination du liquide contenu dans le récipient par des agents pathogènes, des saletés, etc.

Selon une autre caractéristique possible, ledit embout nasal est réalisé dans un matériau élastiquement déformable.

De manière avantageuse, l'embout nasal est réalisé dans un matériau élastiquement déformable, tel que du polypropylène, du polyéthylène, etc., de manière à faciliter l'insertion en force de l'embout de décharge dans le canal dudit embout nasal et sa fixation par frottements et/ou par compression.

Selon une autre caractéristique possible, ledit liquide de nettoyage nasal est du sérum physiologique.

De manière avantageuse, le liquide de nettoyage nasal est une solution isotonique ou hypertonique (tel que du sérum physiologique), ceci afin de ne pas léser ou irriter les muqueuses situées dans la cavité nasale.

Selon une autre caractéristique possible, ledit dispositif d'injection est une seringue, une poire à lavement ou un pot neti.

L'invention concerne également un récipient pour kit pour irrigation nasale, caractérisé en ce que ledit récipient est configuré pour être mis en œuvre dans kit tel que défini ci-dessus.

L'invention se rapporte également à un procédé de préparation d'un dispositif d'injection pour irrigation nasale à partir d'un kit tel que défini ci-dessus, caractérisé en ce que ledit procédé comprend les étapes suivantes :
- introduction de l'embout de décharge du dispositif d'injection dans l'orifice de distribution du récipient de liquide de nettoyage nasal ;
- remplissage du dispositif, par exemple par pression négative et/ou écoulement gravitaire, de liquide de nettoyage nasal ;
- retrait de l'embout de décharge du dispositif d'injection de l'orifice de distribution du récipient de liquide de nettoyage nasal ;
- introduction de l'embout de décharge du dispositif dans une des extrémités du canal de l'embout nasal.

L'invention sera mieux comprise, et d'autres buts, détails, caractéristiques et avantages de celles-ci apparaîtront plus clairement au cours de la description suivante d'un mode de réalisation particulier de l'invention, donnée uniquement à titre illustratif et non limitatif, en référence aux dessins annexés, sur lesquels :
- La [Fig.1] illustre une vue très schématique et en perspective d'un kit pour irrigation nasale selon l'invention ;
- la [Fig. 2] est une vue très schématique agrandie et en coupe d'une partie du récipient du kit de la [Fig. 1] ;
- la [Fig. 3] est une vue très schématique et en perspective d'un opercule du récipient des [Fig. 1] et [Fig. 2] ;
- la [Fig. 4] est une vue très schématique et en perspective d'un bouchon du récipient des [Fig. 1] à [Fig. 3] ;
- la [Fig. 5] est une vue très schématique et en perspective d'un dispositif d'injection sur lequel est monté un embout nasal du kit de la [Fig. 1] ;
- la [Fig. 6] illustre un logigramme des étapes d'un procédé de préparation d'un dispositif d'injection pour irrigation nasale à partir du kit de la [Fig. 1].

Dans la description qui suit, le terme « comprendre » est synonyme de « inclure » et n'est pas limitatif en ce qu'il autorise la présence d'autres éléments dans le kit, ou la structure ou composant auxquels il se rapporte. Il est entendu que le terme « comprendre » inclut les termes « consister en ».

De même, les termes « inférieur », « supérieur », « avant », « arrière », « longitudinal » et « transversal » s'entendront par rapport à l'orientation générale de la structure ou de l'élément considéré. Par ailleurs, on notera que sur les différentes figures, les mêmes références désignent des éléments identiques ou similaires.

Dans la présente demande, on notera qu'on entend par « sensiblement parallèle », une direction s'écartant d'au plus 20°, voire d'au plus 10° ou d'au plus 5° d'une direction parallèle. On notera également qu'on entend par « sensiblement orthogonale », une direction s'écartant d'au plus 20°, voire d'au plus 10° ou d'au plus 5° d'un angle d'une valeur de 90°.

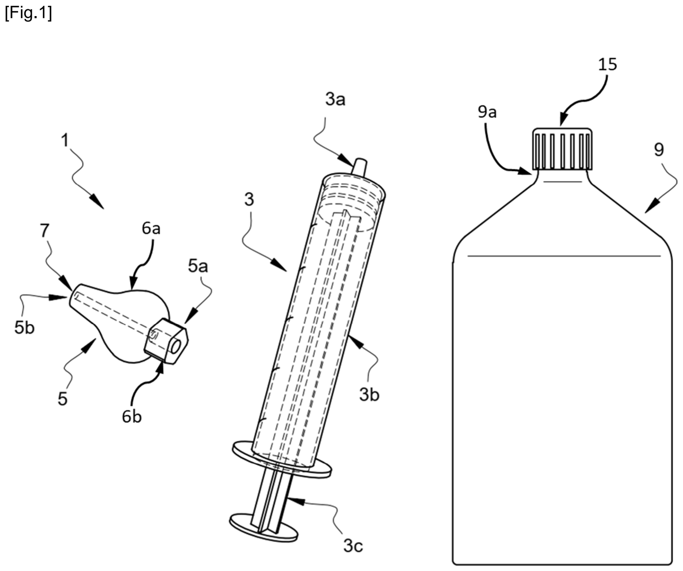

La [Fig. 1] illustre ainsi une vue schématique et perspective des différents éléments d'un kit 1 pour irrigation nasale selon l'invention.

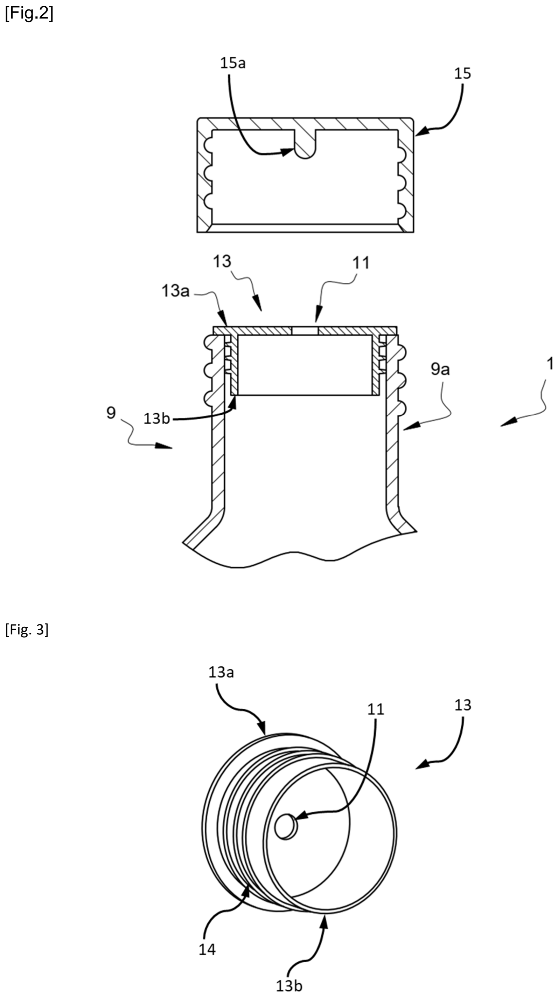

Ledit kit 1 comprend ainsi au moins :
- un dispositif d'injection 3 comprenant un embout 3a de décharge ;
- un embout nasal 5 comprenant un canal 7 débouchant à deux extrémités opposées 5a et 5b dudit embout nasal 5, ledit embout nasal 5 étant configuré pour que l'embout 3a de décharge dudit dispositif d'injection 3 puisse être inséré dans ledit canal 7 ;
- un récipient 9 de liquide de nettoyage nasal qui comprend un orifice de distribution 11 (plus particulièrement visible à la [Fig. 2]).

La [Fig. 2] illustre une vue très schématique, agrandie et partielle de la partie supérieure du récipient 9. Ledit récipient 9 est par un exemple une bouteille ou un flacon présentant un col 9a dans lequel une ouverture est obturée par un opercule 13. Ledit liquide de nettoyage nasal contenu dans le récipient 9 est par exemple du sérum physiologique, une solution saline, etc.

Ledit opercule 13 comprend ainsi une paroi 13a qui obture l'ouverture du col 9a, ledit orifice 11 étant aménagé dans ladite paroi 13a. Ledit orifice 11 de distribution est ainsi aménagé dans ledit opercule 13 (ledit orifice 11 est traversant et s'étend sensiblement orthogonalement au plan d'extension principal de la paroi 13a).

De plus, la paroi 13a présente avantageusement une épaisseur inférieure ou égale à la longueur dudit embout 3a de décharge dudit dispositif d'injection 3. Ledit orifice 11 présente ainsi une longueur d'au moins 0,5 mm, et présente par exemple une longueur comprise entre 0,5 et 10 mm.

On notera qu'on entend par longueur de l'orifice 11, ou encore profondeur de l'orifice 11, la direction (ou dimension) sensiblement orthogonale à la section de passage dudit orifice 11.

La [Fig. 3] est une vue très schématique et en perspective de l'opercule 13 du récipient 9. Ledit opercule 13 comprend ainsi une portion tubulaire 13b dont l'une des extrémités est fermée par la paroi 13a dans laquelle est aménagé l'orifice de distribution 11. Ledit opercule 13 présente ainsi une forme sensiblement cylindrique.

Ainsi, la portion tubulaire 13b est configurée pour s'insérer dans l'ouverture du col 9a et la paroi 13a venir au contact de la partie supérieure du col 9a. Ladite portion tubulaire 13a comprend par exemple un filetage 14, aménagé sur la face externe de la portion tubulaire 13b, filetage qui coopère avec un filetage (ou taraudage) complémentaire (non représenté) aménagé sur la face interne dudit col 9a.

La coopération de la portion tubulaire 13b avec l'ouverture du col 9a, par exemple au moyen de filetages, permet ainsi de fixer l'opercule 13 audit récipient 9, ledit opercule 13 étant avantageusement configuré pour être fixé de manière amovible.

De plus, la coopération des filetages 13 de l'opercule 13 et du récipient 9, ainsi que la coopération de la paroi 13a avec ledit récipient 9 permet avantageusement à l'opercule 13 d'être monté de façon étanche sur ledit récipient 9.

Le dispositif d'injection 3, quant à lui, est par exemple une seringue, ladite seringue comprenant un tube 3b et un piston 3c. Le tube 3b de la seringue 3 comprend ainsi deux extrémités ouvertes opposées, l'une correspondant à l'embout de décharge 3a, tandis que l'autre extrémité est fermée par le piston 3c qui est configuré pour pouvoir coulisser dans ledit tube 3b. Le remplissage du tube 3b de la seringue 3 se fait par l'intermédiaire de l'embout de décharge 3a.

Ledit embout nasal 5, quant à lui, est avantageusement un élément monobloc ou unitaire réalisé en un matériau élastiquement déformable, tel que du polypropylène, du polyéthylène, etc., de manière à faciliter l'insertion en force de l'embout de décharge 3a dans le canal 7 dudit embout nasal 5 et sa fixation par frottements (et/ou par compression des parois délimitant ledit canal autour dudit embout de décharge).

De plus, ledit embout nasal 5 comprend un corps principal 6a, sensiblement en forme de goutte, et une structure sensiblement cylindrique 6b fixée audit corps principal 6a.

Le canal 7 est ainsi aménagé dans ledit embout nasal 5 (et à travers celui-ci), traversant ledit embout nasal 5 d'une extrémité 5a à l'autre 5b. L'extrémité 5a correspond sensiblement à la structure cylindrique 6b, tandis que l'autre extrémité 5b correspond à la forme la plus effilée ou progressive du corps principal 6a.

On notera que la partie 5b la plus effilée ou progressive dudit embout nasal 5 est destinée à être insérée dans une narine de cavité nasale et obturer ainsi au mieux la narine pour éviter que le liquide injecté à travers le canal 7, au moyen du dispositif d'injection 3, ne s'échappe par ladite narine obturée.

Par ailleurs, l'orifice de distribution 11 est configuré, d'une part, pour présenter une coopération de forme avec l'embout de décharge 3a du dispositif d'injection 3, et d'autre part, pour présenter un diamètre inférieur à chacune des extrémités 5a et 5b dudit embout nasal 5 où débouche ledit canal 7.

De plus, ledit embout de décharge 3a dudit dispositif d'injection 3 est configuré pour être emmanché en force dans le canal 7 de l'embout nasal 5 et dans l'orifice de distribution 11 dudit récipient 9.

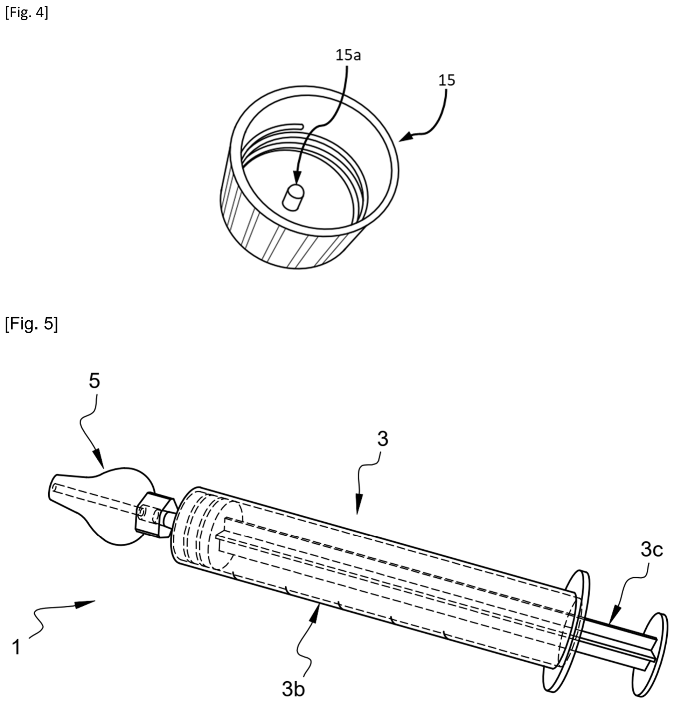

Comme illustré à la [Fig. 4], ledit récipient 9 comprend un bouchon 15 qui est configuré pour être fixé sur le col 9a dudit récipient 9. Ledit bouchon 15 comprend ainsi un pion d'obturation 15a qui est configuré pour obturer l'orifice 11 dudit opercule 13 lorsque le bouchon 15 est fixé au récipient 9 (au niveau du col 9a).

La [Fig. 5] est, quant à elle, une vue très schématique et en perspective du dispositif d'injection 3 sur lequel est monté l'embout nasal 5 du kit 1, ceci après emmanchement en force de l'embout de décharge 3a dans le canal 7. Ainsi, ledit kit 1 selon l'invention permet de procéder à la préparation d'un dispositif d'injection 3 permettant de réaliser une irrigation nasale.

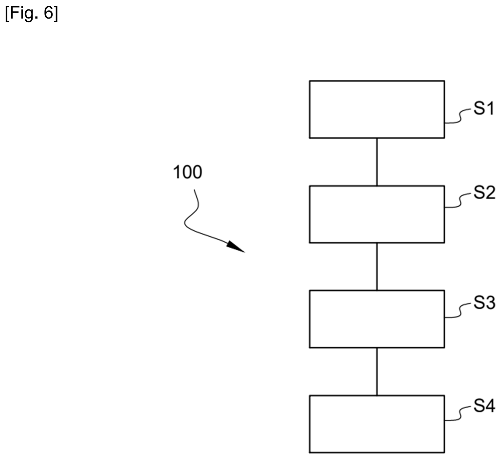

Plus particulièrement, la [Fig. 6] illustre un logigramme d'un procédé 100, selon l'invention, de préparation d'un dispositif d'injection 3 pour irrigation nasale à partir du kit 1, ledit procédé 100 comprenant les étapes suivantes :
- introduction S1 de l'embout de décharge 3a du dispositif d'injection 3 dans l'orifice 11 du récipient 9 de liquide de nettoyage nasal, par exemple par emmanchement en force ;
- remplissage S₂ du dispositif d'injection 3, par exemple par pression négative et/ou par écoulement gravitaire, de liquide de nettoyage nasal ;
- retrait S₃ de l'embout de décharge 3a du dispositif d'injection 3 de l'orifice de distribution 11 du récipient 9 de liquide de nettoyage nasal ;
- introduction S₄ de l'embout de décharge 3a du dispositif dans une des extrémités 5a du canal 7 de l'embout nasal 5, par exemple par emmanchement en force.

Le procédé 100 permet ainsi d'obtenir un dispositif d'injection 3 rempli de liquide nettoyant provenant du récipient 9 et sur lequel est monté l'embout nasal 5, tout en limitant les risques de contamination de l'embout de décharge 3a dudit dispositif 3 et du liquide de nettoyage nasal contenu dans le récipient 9.

Dans des variantes de réalisation non représentées de l'invention, le dispositif d'injection du kit est une poire à lavement ou un pot neti.

Dans une autre variante de réalisation non représentée de l'invention, l'orifice de distribution du récipient présente une section progressive. Plus particulièrement, ledit orifice comprend une section progressive et une section droite prolongeant ladite section progressive, la partie la plus évasée étant orientée vers l'extérieur dudit récipient.

Dans une autre variante de réalisation non représentée de l'invention, ledit embout de décharge dudit dispositif d'injection et l'orifice de distribution dudit récipient comprennent respectivement un taraudage et un filetage complémentaires (ou des filetages complémentaires), lesdits embout de décharge et orifice de distribution étant configurés pour être vissés l'un à l'autre.

## Revendications

1. Kit (1) pour irrigation nasale, ledit kit (1) comprenant :
- un dispositif d'injection (3) comprenant un embout de décharge (3a) ;
- un embout nasal (5) comprenant un canal (7) débouchant à deux extrémités (5a, 5b) opposées dudit embout nasal (5), ledit embout nasal (5) étant configuré pour que l'embout de décharge (3a) dudit dispositif d'injection (3) puisse être inséré dans ledit canal (7) ;
- un récipient (9) de liquide de nettoyage nasal qui comprend un orifice de distribution (11) ;
**caractérisé en ce que** ledit un orifice (11) est configuré, d'une part, pour présenter une coopération de forme avec l'embout de décharge (3a), et d'autre part, pour présenter un diamètre inférieur à chacune des extrémités (5a, 5b) dudit embout nasal (5) où débouche ledit canal (7).

2. Kit (1) selon la revendication précédente, **caractérisé en ce que** ledit récipient (9) comprend un col (9a) ouvert obturé par un opercule (13), ledit orifice de distribution (11) étant aménagé dans ledit opercule (13).

3. Kit (1) selon la revendication précédente, **caractérisé en ce que** ledit opercule (13) comprend une paroi (13a), ledit orifice de distribution (11) étant aménagé dans ladite paroi (13a), la paroi (13a) présentant une épaisseur inférieure ou égale à la longueur dudit embout de décharge (3a) dudit dispositif d'injection (3).

4. Kit (1) selon la revendication précédente, **caractérisé en ce que** ledit orifice (11) présente une longueur d'au moins 0,5 mm, et présente par exemple une longueur comprise entre 0,5 et 10 mm.

5. Kit (1) selon l'une quelconque des revendications précédentes, **caractérisé en ce que** ledit embout de décharge (3a) dudit dispositif d'injection (3) est configuré pour être emmanché en force dans le canal (7) de l'embout nasal (5) et dans l'orifice de distribution (11).

6. Kit (1) selon l'une quelconque des revendications précédentes, **caractérisé en ce que** ledit récipient (9) comprend un bouchon (15) qui est configuré pour être fixé sur le col (9a) dudit récipient (9), ledit bouchon (15) comprenant un pion d'obturation (15a) qui est configuré pour obturer l'orifice de distribution (11) dudit opercule (13) lorsque le bouchon (15) est fixé audit col (9a).

7. Kit (1) selon l'une quelconque des revendications précédentes, **caractérisé en ce que** ledit liquide de nettoyage nasal est du sérum physiologique.

8. Kit (1) selon l'une quelconque des revendications précédentes, **caractérisé en ce que** ledit dispositif d'injection (3) est une seringue (3), une poire à lavement ou un pot neti.

9. Récipient (9) pour kit pour irrigation nasale, **caractérisé en ce que** ledit récipient (9) est configuré pour être mis en œuvre dans un kit (1) selon l'une quelconque des revendications précédentes.

10. Procédé (100) de préparation d'un dispositif d'injection (3) pour irrigation nasale à partir d'un kit (1) selon l'une quelconque des revendications 1 à 8,
**caractérisé en ce que** ledit procédé (100) comprend les étapes suivantes :
- introduction de l'embout de décharge (3a) du dispositif d'injection (3) dans l'orifice de distribution (11) de récipient (9) de liquide de nettoyage nasal ;
- remplissage du dispositif d'injection (3) de liquide de nettoyage nasal ;
- retrait de l'embout de décharge (3a) du dispositif d'injection (3) de l'orifice de distribution (11) du récipient (9) de liquide de nettoyage nasal ;
- introduction de l'embout de décharge (3a) du dispositif d'injection (3) dans une des extrémités du canal (7) de l'embout nasal (5).
